# EUROPEAN PATENT SPECIFICATION

(11) **EP 3 932 308 B1**
(45) Date of publication and mention of the grant of the patent: **03.12.2025**
(21) Application number: 20890797.2
(22) Date of filing: 19.08.2020
(51) Int. Cl.: A61B 5/145, A61B 5/155, A61B 5/00

(54) **BODY ATTACHMENT DEVICE FOR CONTINUOUS GLUCOSE MONITORING**
KÖRPERAUFSATZ ZUR KONTINUIERLICHEN GLUCOSEÜBERWACHUNG
DISPOSITIF DE FIXATION AU CORPS DESTINÉ À LA SURVEILLANCE EN CONTINU DU GLUCOSE

(30) Priority: 21.11.2019 KR 20190150315
(43) Date of publication of application: 05.01.2022
(73) Proprietor: i-Sens, Inc., Seoul 06646 (KR)
(72) Inventor: LEE, Jin Won, Seoul 06646 (KR); PARK, Hyo Seon, Seoul 06646 (KR); LEE, Yun Ho, Seoul 06646 (KR)
(74) Representative: BCKIP Part mbB
(86) International application number: PCT/KR2020/011005
(87) International publication number: WO 2021/101022

(56) References cited:
- EP-A1- 2 393 418
- EP-A2- 2 146 624
- EP-A2- 2 327 984
- WO-A1-2010/091028
- WO-A1-2018/125841
- WO-A1-2019/213319
- JP-A- 2007 060 063
- JP-A- 2013 176 579
- KR-A- 20130 134 076
- KR-A- 20140 066 376
- KR-A- 20170 051 134
- US-A1- 2006 132 283
- US-A1- 2011 006 876

## Description

### TECHNICAL FIELD

The present disclosure generally relates to a body attachable apparatus for continuous blood glucose measurement, more specifically, a body attachable apparatus for continuous blood glucose measurement in which, by inputting a user command for applying operation power of the body attachable apparatus by a light or touch type way, battery power can be easily initiated to be applied and the body attachable apparatus is controlled to be operated not only before the body attachable apparatus is attached to a body but also after the body attachable is attached to the body, and by controlling an operation of the body attachable apparatus with light or touch, when the body attachable apparatus is attached to a portion of the body and is being used, the malfunction of the body attachable apparatus caused by mishandling a physical operation button formed at the body attachable apparatus can be prevented and the operation power can be stably provided.

### BACKGROUND

Diabetes is a chronic medical condition that is common in modern people, and in the Republic of Korea, there are 2 million diabetes patients, about 5% of the total population.

Diabetes occurs when the absolute level of the sugar level in blood is high due to the absolute deficiency or relative insufficiency of insulin, produced by the pancreas, caused by various reasons such as obesity, stress, poor eating habits, and inherited hereditary factors and imbalance regarding glucose in the blood.

Because blood glucose needs to be measured steadily in order to manage diabetes, the needs of devices related to blood glucose measurement are constantly increasing. When diabetes patients strictly manage blood glucose control, various researches confirmed that occurrences of complications of diabetes are significantly reduced. Accordingly, it is important for diabetes patients to regularly measure blood glucose for blood glucose management.

Generally, a blood-collecting type blood glucose measurement device (finger prick method) is mainly used for blood glucose management of diabetes patients, and the blood-collecting type blood glucose measurement device can help the blood glucose management of the diabetes patients, but there is a problem that it is difficult of figuring out a precise blood glucose level because the result shows a result at the time of measurement only. Additionally, as the blood-collecting type blood glucose measurement device requires to collect blood several times in a day in order to measure blood glucose, the burden of finger prick for collecting blood to diabetes patients is a problem.

Diabetics patients generally experience hyperglycemia and hypoglycemia, an emergency may occur in the hypoglycemic conditions, and the patients may become unconscious or die if a hypoglycemic condition lasts for an extended period of time without the supply of sugar. Accordingly, although rapid discovery of the hypoglycemic condition is critically important for diabetics, blood-collecting type glucose monitoring devices intermittently measuring glucose have limited ability to accurately measure blood glucose levels.

To overcome such a drawback of the blood-collecting type glucose monitoring devices, continuous glucose monitoring systems (CGMSs) inserted into the human body to measure a blood glucose level every few minutes have been developed, and therefore easily perform the management of diabetics and responses to an emergency situation.

A continuous glucose monitoring system includes a body attachable device attached to a body part of a user and measuring a blood glucose level by extracting body fluid, a communication terminal outputting the received blood glucose level, and so on. The body attachable device measures the blood glucose of a user in a status that a sensor is inserted to a human body for a certain period, for example, 15 days, and generates blood glucose information periodically. An application for blood glucose management is installed at the communication terminal, and the communication terminal periodically receives and outputs the blood glucose information from the body attachable unit so that the user can check the received blood glucose information.

A sensor unit sensing a blood glucose wherein a portion of the sensor unit is inserted into a human body, a PCB board electrically connected with the sensor unit, calculating a blood glucose level from blood glucose information received from the sensor unit and transmitting the calculated blood glucose value to a communication terminal, and a battery providing operation power to the PCB board are mounted in the inside of the housing of the body attachable apparatus. Various types of electronic components calculating the blood glucose level from the blood glucose information received from the sensor unit, storing the blood glucose level for a certain period, or wirelessly connected with a communication terminal and periodically transmitting the blood glucose level to the communication terminal are installed in the PCB board, and those PCB board and the battery are manufactured as a sensor transmitter and the sensor transmitter and the sensor unit coupled to each other consist the body attachable apparatus.

The body attachable apparatus is replaced with a new body attachable apparatus after a certain use period, for example, after it has been used for a month, and the battery of the body attachable apparatus has limited power and therefore the operation power needs to be provided through the battery to operate the body attachable apparatus after the body attachable apparatus is disposed on the human body.

When the sensor transmitter and the sensor unit are manufactured as a separate type, each of the sensor transmitter and the sensor unit has a connection terminal or socket, and when the sensor transmitter and the sensor unit are electrically coupled to each other through the connection terminal or socket, the operation is initiated by providing the operation power to the PCB board and the sensor unit.

When the sensor transmitter and the sensor unit are manufactured as an integrated type, a user needs to initiate the operation of the body attachable apparatus by himself or herself after or just before the body attachable apparatus is positioned to the human body, and a separate operation button is formed on a housing outside of a conventional body attachable apparatus and the operation power of the battery is supplied to the PCB board or the sensor unit through the operation button and therefore the manipulation of the button in a state that the body attachable apparatus is attached to the human body is inconvenient.

Furthermore, because a physical manipulation button needs to be formed at the body attachable apparatus and a configuration for electrically connecting between the battery and the PCB board through the manipulation button needs to be added, there is a problem having difficulty in minimizing and manufacturing the body attachable apparatus.

Additionally, there is a problem that because the battery and the PCB board are electrically connected by a physical manipulation button, battery power can be discharged due to the malfunction of the manipulation button before the body attachable unit contacts the skin, or the operation power cannot be stably provided due to the malfunction of the manipulation button after the body attachable unit contacts the skin. Prior art measurement devices are disclosed in WO 2018/125841 A1, WO 2019/213319 A1 and WO 2010/091028 A1.

### SUMMARY

### Technical Problem

The present disclosure is invented to solve problems in conventional technique of a body attachable device for continuous blood glucose measurement discussed above, and the purpose of the present disclosure is for providing a body attachable apparatus for continuous blood glucose measurement providing operation power by easily initiating operation according to a connection command of a light input, a touch input or the like.

Another purpose of the present disclosure is for providing a body attachable unit for continuous blood glucose measurement which can be manufactured to minimalize its size by controlling electric connection between a battery and a PCB board through a light input or a touch input instead of a physical manipulation button to a body attachable apparatus.

Still another purpose of the present disclosure is for providing a body attachable unit for continuous blood glucose measurement stably providing operation power by controlling electric connect between a battery and a PCB board through a light input or a touch input.

### Solution to Problem

According to the present invention, a body attachable apparatus for continuous blood glucose measurement continuously measuring a blood glucose value by being attached to a body of a user may comprise: a housing; a sensor unit, wherein a portion of the sensor unit is inserted to a body of the user to continuously measure a blood glucose of a user; a printed circuit board (PCB) disposed inside the housing and configured to be electrically connected to the sensor unit; a battery disposed inside the housing and configured to supply operation power needed for the PCB or the sensor unit; and a connection control unit configured to control electric connection of the battery and the PCB.

Here, the connection control unit comprises:
a control command input unit to which a connection command is inputted; a signal generating unit configured to generate a connection control signal based on the inputted connection command; and a switch unit electrically connecting between the battery and the PCB according the connection control signal.

According to one embodiment of the present disclosure, the control command input unit comprises a touch panel, and the signal generating unit is configured to, when the connection command of the user is inputted through the touch panel, generate the connection control signal.

According to one embodiment of the present disclosure, the signal generating unit is configured to compare a pattern of the connection command inputted through the touch panel with a reference set pattern, and when the pattern of the connection command is identical to the reference set pattern, generate the connection control signal.

According to the present invention, the connection control unit is configured to receive the connection control signal, and further comprises a second switch unit configured to block power of the battery supplied to the control command input unit and the signal generating unit based on the connection control signal.

According to another embodiment of the present disclosure, the control command input unit comprises an optical receiver, and the signal generating unit is configured to, when the connection command of the user is inputted through the optical receiver, generate the connection control signal.

According to another embodiment of the present disclosure, the signal generating unit is configured to compare a pattern of the connection command inputted through the optical receiver with a reference set pattern, and when the pattern of the connection command is identical to the reference set pattern, generate the connection control signal.

According to another embodiment of the present disclosure, the signal generating unit is configured to generate the connection control signal by comparing a light frequency and pattern of the connection command inputted through the optical receiver with a light frequency and pattern of the reference set pattern.

According to another embodiment of the present disclosure, the connection control unit is configured to receive the connection control signal, and further comprises a second switch unit configured to block power of the battery supplied to the optical receiver and the signal generating unit based on the connection control signal.

The signal generating unit according to an embodiment of the present disclosure is configured to search for a reference set pattern identical to a pattern of the inputted connection command, and generate the connection control signal corresponding to the searched reference set pattern.

Preferably, the connection control signal generated by the signal generating unit according to an embodiment of the present disclosure is a connection signal for electrically connecting between the battery and the PCB through the switch unit or a block signal for blocking electric connection between the battery and the PCB.

A body attachable apparatus for continuous blood glucose measurement may further comprise a blocking film blocking inputting the connection command to the control command input unit, wherein the blocking film is removably mounted to the control command input unit.

### Advantageous Effects of Invention

A body attachable apparatus for continuous blood glucose measurement according to various embodiments of the present disclosure may have following technical effects.

Firstly, by inputting a user command for applying operation power of a body attachable apparatus by a type way of light, touch or the like, a body attachable apparatus for continuous blood glucose measurement according to an embodiment of the present disclosure can be controlled to operate to easily initiate to apply battery power not only before the body attachable apparatus is attached to a body but also after the body attachable is attached to the body.

Secondly, a body attachable apparatus for continuous blood glucose measurement according to an embodiment of the present disclosure does not use a physical manipulation button or a physical apparatus for connecting between a battery and a PCB board through a physical manipulation button but is manufactured to apply battery power to the PCB board according to a connection command inputted with a type of light or touch, and therefore the size of the body attachable apparatus can be minimalized.

Thirdly, a body attachable apparatus for continuous blood glucose measurement according to an embodiment of the present disclosure controls the operation of the body attachable apparatus by light or touch, and therefore the malfunction of the body attachable apparatus caused by mistakenly touching a physical manipulation button formed at the body attachable apparatus while a user is using the body attachable apparatus can be prevented and operation power can be stably supplied.

### BRIEF DESCRIPTION OF THE DRAWINGS

FIG. 1 is a drawing for illustrating an overall system inserting a body attachable apparatus to a human body using an applicator.
FIG. 2 illustrates an exploded perspective view of a body attachable apparatus for continuous blood glucose measurement according to an embodiment of the present disclosure.
FIG. 3 is a block diagram for illustrating a body attachable apparatus for continuous blood glucose measurement according to an embodiment of the present disclosure.
FIG. 4 is a block diagram of a connection control unit according to an embodiment of the present disclosure.
FIG. 5 is a flow chart for illustrating a method of controlling supply of battery power at a connection control unit according to an embodiment of the present disclosure.
FIG. 6 is a flow chart for illustrating a method of controlling supply of battery power at a connection control unit according to another embodiment of the present disclosure.
FIG. 7 is a flow chart for illustrating a method of controlling supply of battery power at a connection control unit according to still another embodiment of the present disclosure.

### DETAILED DESCRIPTION OF EMBODIMENTS

The technical terms used in the present disclosure are only for the purpose of describing exemplary embodiments, and they are not intended to limit the present invention. Also, unless otherwise defined, all technical terms used herein should be construed as having the same meaning as commonly understood by those skilled in the art, and should not be interpreted as being excessively inclusive or excessively restrictive. In addition, when a technical term used herein is an erroneous technical term that does not accurately represent the present invention, it should be understood as replacing the term by a technical term which can be properly understood by those skilled in the art.

Additionally, singular expressions used in the present disclosure include plural meaning unless the context clearly dictates otherwise. In the present disclosure, the terms "comprising", "having", "including" and the like should not be construed to necessarily include all component elements or steps described in the present disclosure, and it should be interpreted that some component elements or some steps among them may not be included, or other components or steps may be added thereto.

Also, it should be noted that the accompanying drawings are merely illustrated to easily explain the invention, and therefore, they should not be construed to limit the invention.

With reference to the accompanying drawings, a body attachable apparatus for continuous blood glucose measurement according to an embodiment of the present disclosure is described as follows.

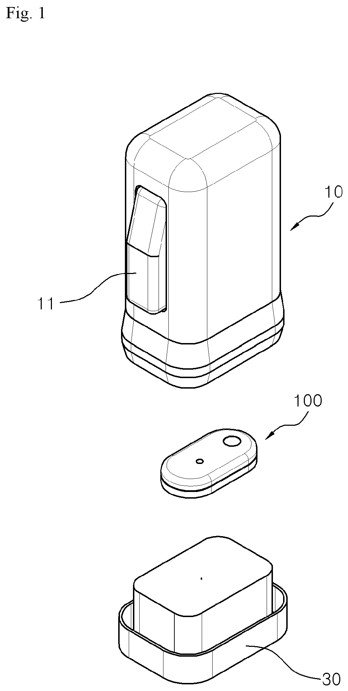

FIG. 1 is a drawing for illustrating an overall system inserting a body attachable apparatus to a human body using an applicator.

As illustrated in FIG. 1, a body attachable apparatus (100) is attached to a human body by an applicator (10), the applicator (10) is formed so that the body attachable apparatus (100) can be fixedly coupled to an inside of the applicator (10) and the applicator (10) is operated such that the body attachable apparatus (100) can be outwardly discharged by manipulation of an user.

In this embodiment, the body attachable apparatus (100) is manufactured in a state that body attachable apparatus (100) is inserted into the inside of the applicator (10), and the body attachable apparatus (100) which is disposed inside the applicator (10) is configured to be discharged according to manipulation of a shooting button (11) by a user so that the body attachable apparatus (100) can be moved in an outwardly discharging direction and be attached to a human body.

A separate protection cap (30) can be separatably coupled to the applicator (10) so that the body attachable apparatus (100) is not exposed to the outside of the applicator (10), and only after the protection cap (30) is separated, the user can attach the body attachable unit apparatus (100) to the human body by manipulating the applicator (10).

More specifically, the applicator (10) is configured that in a state that the body attachable apparatus (100) is inserted in the applicator (10) the applicator (10) fixes the body attachable apparatus (100) therein and in a state that the body attachable apparatus (100) is moved and discharged to the outside of the applicator (10) the fixation state with respect to the body attachable apparatus (100) is released. Accordingly, in a state in which the body attachable apparatus (100) is assembled to be inserted into the inside of the applicator (10) the body attachable apparatus (100) maintains a fixed state, and when the body attachable apparatus (100) is outwardly discharged and attached to the skin by manipulating the applicator (10) the fixed state in which the body attachable apparatus (100) is coupled and fixed to the applicator (10) is released and therefore when the applicator (10) is separated in this state the applicator (10) is separated from the body attachable apparatus (100) and only the body attachable apparatus (100) remains in a state that is attached to the skin.

When the body attachable apparatus (100) is attached to the skin, a sensor unit of which one end portion is inserted into the human body, a wireless communication chip configured to wirelessly communicate with an external terminal, and so on are disposed inside the body attachable apparatus (100).

The body attachable apparatus for continuous blood glucose measurement extracts body fluid and periodically measures blood glucose and transmits the measured blood glucose value to the external terminal (not shown) like a smartphone through the wireless communication chip.

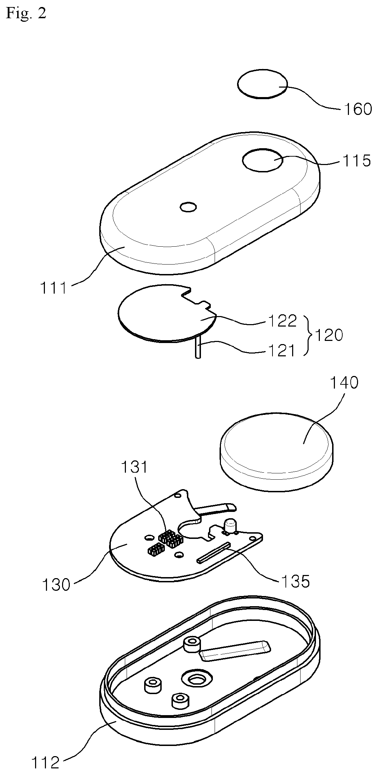

FIG. 2 illustrates an exploded perspective view of a body attachable apparatus for continuous blood glucose measurement according to an embodiment of the present disclosure.

Referring to FIG. 2, the body attachable apparatus (100) for continuous blood glucose measurement is configured to comprise a housing on which an adhesive tape (not shown) is disposed so that the bottom surface can be attached to the skin, a sensor unit (120) which is disposed inside the housing such that one end portion of the sensor unit (120) outwardly protrudes from the bottom surface of the housing and, when the housing is attached to the skin, one end portion of the sensor unit (120) is inserted into the human body, a PCB board (130) disposed in the inside of the housing, a battery (140) supplying operation power to the body attachable unit, and a connection control unit configured to control to apply the power of the battery (140) to the PCB board (130).

One end portion of the sensor unit (120) is formed to be inserted into the human body and the other end portion of the sensor unit (120) is formed to contact the PCB board (130) or be connected to the PCB board (130), a sensor body unit (122) is formed to contact an electric contact point of the PCB board (130) at the other end portion of the sensor unit (120), and a sensor probe unit (121) which is formed to extend in a form bent from one side of the sensor body unit (122) and outwardly protrudes from the housing so that it can be inserted into the human body is formed at one end portion of the sensor unit (120). The sensor body unit (122) is formed to have a structure having a relatively large area and the sensor probe unit (121) is formed to have a relatively narrow and long structure, but according to a field to which the present disclosure is applied a shape of the sensor body unit (122) and the sensor probe unit (121) can be varied.

The housing is formed to be separatable into an upper housing (111) and a lower housing (112) and to have an inner accommodating space, a sensor guide unit (not shown) which can support and guide a partial portion of the sensor probe unit (121) is formed at the inside of the housing, and a board supporting unit (not shown) fixing and supporting the PCB board (130) to a certain position may be formed at the inside of the housing.

An electric connection point (131) is formed at the PCB board (130) to be electrically connected with the sensor unit (120), and a wireless communication chip (135) and so on are mounted to the PCB board (130) to transmit the blood glucose measurement result measured by the sensor unit (120) to the external terminal. The embodiment of the present disclosure have the wireless communication chip (135) inside the body attachable unit (100) and therefore can easily communicate with the external terminal without a separate operation of transmitter connection.

Additionally, a battery (140) is mounted to the inside of the housing to supply the power to the PCB board (130), and in the exemplary embodiment, the battery (140) may be arranged such that the battery (140) is mounted to one surface of the PCB board (130) or the battery (140) is disposed at an independent or separate area from the PCB board (130). Areas of the PCB board (130) and the battery (140) projected to the bottom surface of the housing are independently arranged without an overlapped area, and therefore the thickness of the body attachable unit (100) can be reduced and its size can be more minimized.

In this embodiment, the housing may further separately include a connection control unit providing or blocking the power of the battery (140) to or from the PCB board (130) according to an external user manipulation.

A connection control unit according to an embodiment of the present disclosure comprises a signal generating unit and a switching element or a switch providing or blocking the power of the battery (140) to or from the PCB board (130) according to a connection control signal generated by the signal generating unit, and when the signal generating unit generates the connection control signal according to the manipulation of the user, the switching element electrically connects between the PCB board (130) and the battery (140) according to the generated connection control signal, and thereby the power necessary for operating the body attachable apparatus is supplied from the battery (140) to the PCB board (130) so that the sensor unit (120), the wireless communication chip (135) and other elements can initiate to operate.

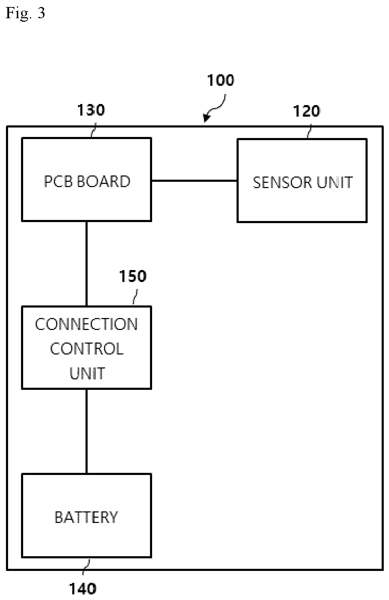

FIG. 3 is a block diagram for illustrating a body attachable apparatus for continuous blood glucose measurement according to an embodiment of the present disclosure.

Referring to FIG. 3, the sensor unit (120) and the PCB board (130) are electrically connected with each other, and a portion of the sensing unit (120) is inserted into the human body to periodically extract body fluid and generate blood glucose information of the user from the extracted body fluid, and the PCB board (130) calculates a blood glucose value based on the blood glucose information received from the sensor unit (120) and transmits the calculated glucose value to an external terminal periodically or in real time.

The operation of the sensor unit (120) and the PCB board (130) is performed in a state that after the body attachable apparatus is attached to the human body the body attachable apparatus initiates to operate by the supply of the power of the battery (140), when the body attachable apparatus is disposed inside the applicator and therefore is in a unused state, the supply of the power of the battery (140) to the sensor unit (120) or the PCB board (130) needs to be blocked in order to prevent consuming the limited power of the battery (140) with by the sensor unit (120) or the PCB board (130), and the power of the battery (140) needs to be supplied to the sensor unit (120) or the PCB board (130) after the body attachable apparatus is attached to the human body.

The connection control unit (150) comprises a switching element or a switch providing or blocking the power of the battery (140) to or from the PCB board (130) or the sensor unit (120) according to the inputted connection command of the user, and the connection control unit (150) generates a connection control signal for controlling the operation of the switching element according the user control command and the switching element electrically connects between the battery (140) and the PCB board (130) or blocks electric connection between the battery (140) and the PCB board (130) and provides or blocks the power of the battery (140) supplied to the PCB board (130).

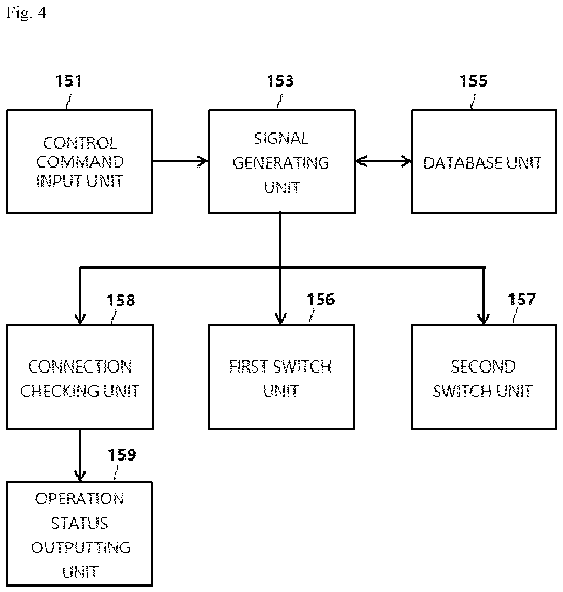

FIG. 4 is a block diagram of a connection control unit according to an embodiment of the present disclosure.

Referring to FIG. 4, a control command input unit (151) is mounted to a housing outside of the body attachable apparatus and is configured to receive a connection command of the user from an external device. For example, the control command input unit (151) can be implemented with an optical receiver, a touch panel and the like, and the user can input the connection command to the optical receiver by using a light emitter or touch the touch panel to input the connection command.

A signal generating unit (153) generates a connection control signal for controlling an operation of a first switching unit (156) according to a connection command, and a switch of the first switch unit (156) is turned on or off according to the connection control signal to supply or block the battery power to or from components of the body attachable apparatus, such as the PCB board or the sensor unit, which need the power.

Preferably, the signal generating unit (153) searches for a reference set pattern corresponding to or matching a connection control pattern at a database unit (155), and if the reference set pattern corresponding to or matching the connection control pattern is found at the database unit (155), a connection control signal is generated to turn on the first switch unit (156).

More preferably, the signal generating unit (153) searches for a reference set pattern corresponding to or matching a connection control pattern, and if the reference set pattern corresponding to or matching the connection control pattern is found at the database unit (155), a connection control signal corresponding to the reference set pattern is generated. For example, when the connection control pattern corresponds to a reference set pattern for controlling the first switch to be turned on a connection signal is generated, and when the connection control pattern corresponds to a reference set pattern for controlling the first switch to be turned off a connection block signal is generated.

When the connection control signal is generated from the signal generating unit (153), a second switch unit (157) receives the connection control signal from the signal generating unit (153). Preferably, when the second switch unit (157) receives the connection control signal, the power supplied from the battery to the control command input unit (151) is blocked.

After a connection command is inputted to the control command input unit (151) as a first input and a connection control signal is generated according to the connection command to turn on the first switch unit (156), the signal generating unit (153) maintains the on status of the first switch unit (156) by generating the connection control signal. Because generally the body attachable apparatus persistently or continuously operates for a certain user period after operation initiation, there is no need to input an additional connection command and it is possible to occur the malfunction of the body attachable apparatus by the input of an incorrect or wrong connection command or mistake. Accordingly, when the second switch unit (157) receives the connection control signal, the power supplied from the battery to the control command input unit (151) is blocked to prevent the waste of the battery power and the malfunction of the body attachable apparatus caused by the incorrect or wrong connection command or mistake.

Meanwhile, a connection checking unit (158) determines whether the connection control signal is generated from the signal generating unit (153) according to the connection command of the user or determines whether the first switch unit (156) is controlled to be turned on and the battery power is being supplied to the PCB board or the sensor unit, and generates a connection check signal based on the determined result. An operation status outputting unit (159) outputs to the user whether the battery power is applied or supplied to the PCB board or the sensor unit based on the connection check signal. For example, the operation status outputting unit (159) may be implemented as a LED element, a speaker or so on and the supply status of the battery power can be outputted through the LED element or the supply status of the battery power can be outputted through the speaker.

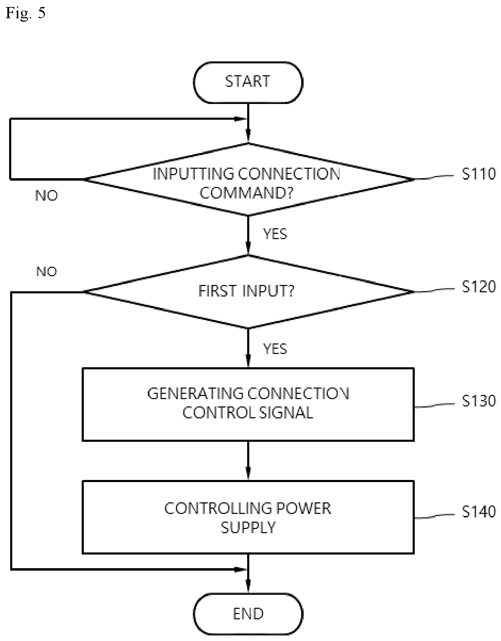

FIG. 5 is a flow chart for illustrating a method of controlling supply of battery power at a connection control unit according to an embodiment of the present disclosure.

Referring to FIG. 5, whether a user connection command for initiating to operate a body attachable apparatus is inputted from an outside is determined (S110).

When the connection command of a user is inputted, whether that connection command is a first inputted connection command is determined (S120). When the connection command is a first inputted connection command, a connection control signal is generated (S130), and the battery power is controlled to be supplied to the PCB board or the sensor unit by controlling the first switch unit to be turned on according to the generated connection control signal (S140).

Meanwhile, when the inputted connection command of the user is not a first inputted connection command, an additionally inputted command is ignored and a connection control signal is not generated.

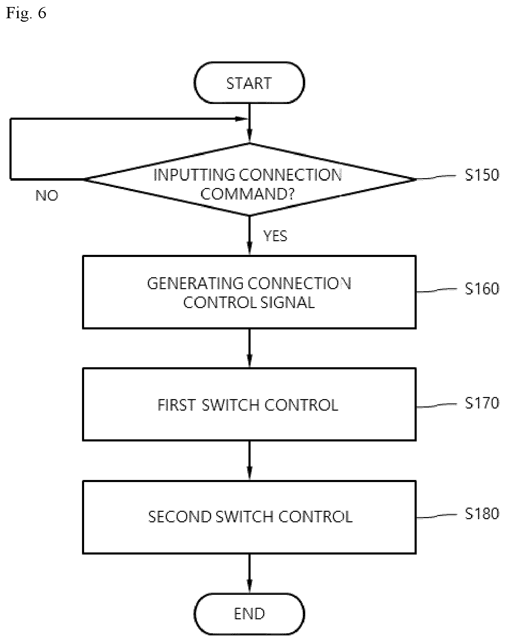

FIG. 6 is a flow chart for illustrating a method of controlling supply of battery power at a connection control unit according to another embodiment of the present disclosure.

Referring to FIG. 6, whether a user connection command for initiating to operate a body attachable apparatus is inputted from an outside is determined (S150).

When the connection command of the user is inputted, a connection control signal is generated (S160). The battery power is controlled to be supplied to the PCB board or the sensor unit by controlling the first switch unit to be turned on based on the connection control signal (S170), and the supply of the battery power to the control command input unit is blocked by controlling the second switch unit to be turned off according to the connection control signal (S180).

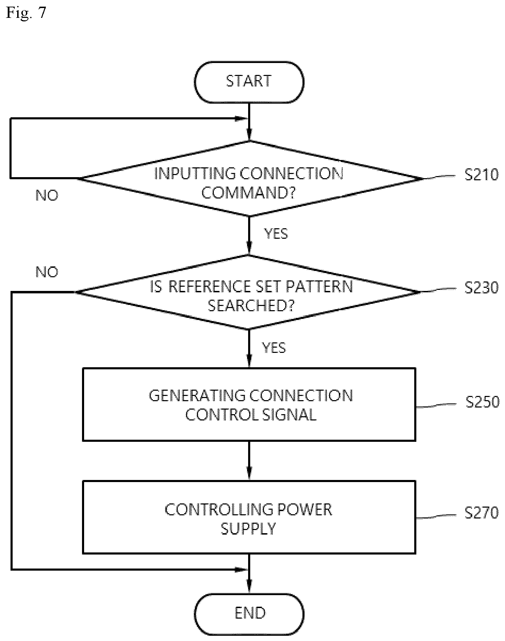

FIG. 7 is a flow chart for illustrating a method of controlling supply of battery power at a connection control unit according to still another embodiment of the present disclosure.

Referring to FIG. 7, whether an user connection command for initiating to operate a body attachable apparatus is inputted from an outside is determined (S210).

When the connection command of the user is inputted, by comparing a connection command pattern and a reference set pattern stored at a database unit, whether a reference set pattern corresponding to or matching the connection command pattern exists is searched (S230).

When a reference set pattern corresponding to or matching the connection command pattern exists, a connection control signal is generated (S250), and power supplied from the battery to the PCB board or the sensor unit is controlled by controlling the operation of the first switch unit according to the connection control signal (S270).

For example, when a reference set pattern corresponding to or matching the connection command pattern exists, a connection control signal is generated and the first switch unit is controlled to be turned on according to the generated connection control signal.

In another example, when a reference set pattern corresponding to or matching the connection command pattern exists, the operation of the first switch unit can be controlled by generating a connection control signal corresponding to a connection command pattern. While the body attachable apparatus is being operating, the operation of the body attachable apparatus can be temporarily halted or stopped if necessary, and a connection signal of controlling the operation of the first switch unit to be turned on according to the connection command pattern can be generated or a connection block signal of controlling the operation of the first switch unit to be turned off according to the connection command pattern can be generated.

Here, when the connection command is inputted in a form of light, a connection command pattern can be expressed with a wavelength of light or time duration of light, cycle and so on, and when light does not correspond to or match a connection command by comparing the connection command pattern with a reference set pattern, the malfunction of the first switch unit can be prevented by filtering such a connection command, or various types of connection control signals such as a connection signal or a connection block signal can be generated.

Meanwhile, when a touch is inputted as a connection command, a connection command pattern is expressed with a touch time, a touch pattern and so on, and when a touch does not correspond to or match a connection command by comparing the connection command pattern with a reference set pattern, the malfunction of the first switch unit can be prevented by filtering such a connection command, or various types of connection control signals such as a connection signal or a connection block signal can be generated.

Meanwhile, the exemplary embodiments of the present disclosure described above can be implemented through programs executable at computers, and can be operated in a general-purpose digital computer executing the programs using computer readable medium.

The above-referenced computer readable medium comprises storage medium such as magnetic storage media (e.g., ROM, floppy disks, hard disks, etc.), optical recording media (e.g., CD-ROMs, DVDs, etc.), and carrier waves (e.g., transmission through the Internet).

Although the present disclosure is described with reference to embodiments shown in the drawings in order to explain certain principles of the present disclosure by way of example, a person having ordinary skill in the art which the present disclosure relates could make various modifications and equivalent other embodiments. The scope of the present invention shall be defined by the claims attached hereto.

## Claims

1. A body attachable apparatus for continuous blood glucose measurement continuously measuring a blood glucose value by being attached to a body of a user, the body attachable apparatus comprising:
a) a housing (111, 112);
b) a sensor unit (120), wherein a portion of the sensor unit (120) is inserted to a body of the user to continuously measure a blood glucose of a user;
c) a printed circuit board (130), PCB, disposed inside the housing (111, 112) and configured to be electrically connected to the sensor unit (120);
d) a battery (140) disposed inside the housing (111, 112) and configured to supply operation power needed for the PCB or the sensor unit (120); and
e) a connection control unit (150) configured to control electric connection of the battery (140) and the PCB;
wherein the connection control unit (150) comprises:
f1) a control command input unit (151) to which a connection command is inputted;
f2) a signal generating unit (153) configured to generate a connection control signal based on the inputted connection command;
f3) a first switch unit (156) electrically connecting (S170) between the battery (140) and the PCB, wherein the first switch unit (156) is configured to be turned on based on the connection control signal to supply battery power to the PCB; and
g) a second switch unit (157) configured to block (S180) power of the battery (140) supplied to the control command input unit (151) and the signal generating unit (153) upon receiving the connection control signal.

2. The body attachable apparatus for continuous blood glucose measurement according to claim 1, wherein:
the control command input unit (151) comprises a touch panel, and
the signal generating unit (153) is configured to, when the connection command of the user is inputted through the touch panel, generate the connection control signal.

3. The body attachable apparatus for continuous blood glucose measurement according to claim 2, wherein the signal generating unit (153) is configured to compare a pattern of the connection command inputted through the touch panel with a reference set pattern, and when the pattern of the connection command is identical to the reference set pattern, generate the connection control signal.

4. The body attachable apparatus for continuous blood glucose measurement according to claim 1, wherein:
the control command input unit (151) comprises an optical receiver, and
the signal generating unit (153) is configured to, when the connection command of the user is inputted through the optical receiver, generate the connection control signal.

5. The body attachable apparatus for continuous blood glucose measurement according to claim 4, wherein the signal generating unit (153) is configured to compare a pattern of the connection command inputted through the optical receiver with a reference set pattern, and when the pattern of the connection command is identical to the reference set pattern, generate the connection control signal

6. The body attachable apparatus for continuous blood glucose measurement according to claim 5, wherein the signal generating unit (153) is configured to generate the connection control signal by comparing a light frequency and pattern of the connection command inputted through the optical receiver with a light frequency and pattern of the reference set pattern.

7. The body attachable apparatus for continuous blood glucose measurement according to claim 2 or 4, wherein the signal generating unit (153) is configured to search for a reference set pattern identical to a pattern of the inputted connection command, and generate the connection control signal corresponding to the searched reference set pattern.

8. The body attachable apparatus for continuous blood glucose measurement according to claim 7, wherein the connection control signal generated by the signal generating unit (153) is a connection signal for electrically connecting between the battery (140) and the PCB through the switch unit or a block signal for blocking electric connection between the battery (140) and the PCB.

9. The body attachable apparatus for continuous blood glucose measurement according to claim 2 or 4, further comprising a blocking film blocking inputting the connection command to the control command input unit (151), wherein the blocking film is removably mounted to the control command input unit (151).

## Patentansprüche

1. Am Körper anbringbare Vorrichtung zur kontinuierlichen Blutzuckermessung, die kontinuierlich einen Blutzuckerwert misst, indem sie an einem Körper eines Benutzers angebracht wird, wobei die am Körper anbringbare Vorrichtung umfasst:
a) ein Gehäuse (111, 112);
b) eine Sensoreinheit (120), wobei ein Abschnitt der Sensoreinheit (120) in einen Körper des Benutzers eingeführt wird, um kontinuierlich einen Blutzucker eines Benutzers zu messen;
c) eine Leiterplatte (130), PCB, die innerhalb des Gehäuses (111, 112) angeordnet ist und konfiguriert ist, um elektrisch mit der Sensoreinheit (120) verbunden zu werden;
d) eine Batterie (140), die innerhalb des Gehäuses (111, 112) angeordnet ist und konfiguriert ist, um Betriebsenergie zuzuführen, die für die PCB oder die Sensoreinheit (120) benötigt wird; und
e) eine Verbindungssteuereinheit (150), die konfiguriert ist, um eine elektrische Verbindung der Batterie (140) und der PCB zu steuern;
wobei die Verbindungssteuereinheit (150) umfasst:
f1) eine Steuerbefehlseingabeeinheit (151), in die ein Verbindungsbefehl eingegeben wird;
f2) eine Signalerzeugungseinheit (153), die konfiguriert ist, um ein Verbindungssteuersignal basierend auf dem eingegebenen Verbindungsbefehl zu erzeugen;
f3) eine erste Schalteinheit (156), die die Batterie (140) und die PCB elektrisch verbindet (S170), wobei die erste Schalteinheit (156) konfiguriert ist, um basierend auf dem Verbindungssteuersignal eingeschaltet zu werden, um der PCB Batterieenergie zuzuführen; und
g) eine zweite Schalteinheit (157), die konfiguriert ist, um Energie der Batterie (140), die der Steuerbefehlseingabeeinheit (151) und der Signalerzeugungseinheit (153) zugeführt wird, bei Empfang des Verbindungssteuersignals zu blockieren (S180).

2. Am Körper anbringbare Vorrichtung zur kontinuierlichen Blutzuckermessung nach Anspruch 1, wobei:
die Steuerbefehlseingabeeinheit (151) ein Berührungsfeld umfasst, und
die Signalerzeugungseinheit (153) konfiguriert ist, um, wenn der Verbindungsbefehl des Benutzers durch das Berührungsfeld eingegeben wird, das Verbindungssteuersignal zu erzeugen.

3. Am Körper anbringbare Vorrichtung zur kontinuierlichen Blutzuckermessung nach Anspruch 2, wobei die Signalerzeugungseinheit (153) konfiguriert ist, um ein Muster des durch das Berührungsfeld eingegebenen Verbindungsbefehls mit einem Referenzsatzmuster zu vergleichen, und wenn das Muster des Verbindungsbefehls mit dem Referenzsatzmuster identisch ist, das Verbindungssteuersignal zu erzeugen.

4. Am Körper anbringbare Vorrichtung zur kontinuierlichen Blutzuckermessung nach Anspruch 1, wobei:
die Steuerbefehlseingabeeinheit (151) einen optischen Empfänger umfasst, und
die Signalerzeugungseinheit (153) konfiguriert ist, um, wenn der Verbindungsbefehl des Benutzers durch den optischen Empfänger eingegeben wird, das Verbindungssteuersignal zu erzeugen.

5. Am Körper anbringbare Vorrichtung zur kontinuierlichen Blutzuckermessung nach Anspruch 4, wobei die Signalerzeugungseinheit (153) konfiguriert ist, um ein Muster des durch den optischen Empfänger eingegebenen Verbindungsbefehls mit einem Referenzsatzmuster zu vergleichen, und wenn das Muster des Verbindungsbefehls mit dem Referenzsatzmuster identisch ist, das Verbindungssteuersignal zu erzeugen.

6. Am Körper anbringbare Vorrichtung zur kontinuierlichen Blutzuckermessung nach Anspruch 5, wobei die Signalerzeugungseinheit (153) konfiguriert ist, um das Verbindungssteuersignal durch Vergleichen einer Lichtfrequenz und eines Musters des durch den optischen Empfänger eingegebenen Verbindungsbefehls mit einer Lichtfrequenz und einem Muster des Referenzsatzmusters zu erzeugen.

7. Am Körper anbringbare Vorrichtung zur kontinuierlichen Blutzuckermessung nach Anspruch 2 oder 4, wobei die Signalerzeugungseinheit (153) konfiguriert ist, um nach einem Referenzsatzmuster zu suchen, das mit einem Muster des eingegebenen Verbindungsbefehls identisch ist, und das Verbindungssteuersignal zu erzeugen, das dem gesuchten Referenzsatzmuster entspricht.

8. Am Körper anbringbare Vorrichtung zur kontinuierlichen Blutzuckermessung nach Anspruch 7, wobei das durch die Signalerzeugungseinheit (153) erzeugte Verbindungssteuersignal ein Verbindungssignal zum elektrischen Verbinden zwischen der Batterie (140) und der PCB durch die Schalteinheit oder ein Blockiersignal zum Blockieren einer elektrischen Verbindung zwischen der Batterie (140) und der PCB ist.

9. Am Körper anbringbare Vorrichtung zur kontinuierlichen Blutzuckermessung nach Anspruch 2 oder 4, ferner umfassend einen Blockierfilm, der die Eingabe des Verbindungsbefehls in die Steuerbefehlseingabeeinheit (151) blockiert, wobei der Blockierfilm abnehmbar an der Steuerbefehlseingabeeinheit (151) montiert ist.

## Revendications

1. Dispositif à fixation corporelle pour une mesure continue de glycémie, mesurant en continu une valeur de glycémie en étant fixé sur le corps d'un utilisateur, ledit dispositif à fixation corporelle comprenant :
a) un boîtier (111, 112) ;
b) une unité de capteur (120), une partie de ladite unité de capteur (120) étant insérée dans le corps de l'utilisateur pour mesurer en continu la glycémie d'un utilisateur ;
c) une carte à circuit imprimé (130), PCB, disposée à l'intérieur du boîtier (111, 112) et prévue pour être connectée électriquement à l'unité de capteur (120) ;
d) une pile (140) disposée à l'intérieur du boîtier (111, 112) et prévue pour fournir l'énergie de fonctionnement nécessaire pour la PCB ou l'unité de capteur (120) ; et
e) une unité de commande de connexion (150) prévue pour commander la connexion électrique de la pile (140) et de la PCB ;
ladite unité de commande de connexion (150) comprenant :
f1) une unité d'entrée d'instruction de commande (151) sur laquelle une instruction de connexion est entrée ;
f2) une unité de génération de signal (153) prévue pour générer un signal de commande de connexion sur la base de l'instruction de connexion entrée ;
f3) une première unité de commutation (156) reliant électriquement (S170) la pile (140) et la PCB, ladite première unité de commutation (156) étant prévue pour être activée sur la base du signal de commande de connexion afin d'alimenter la PCB en énergie de la pile ; et
g) une deuxième unité de commutation (157) prévue pour interrompre (S180) l'alimentation de l'unité d'entrée d'instruction de commande (151) et de l'unité de génération de signal (153) par la pile (140) à la réception du signal de commande de connexion.

2. Dispositif à fixation corporelle pour une mesure continue de glycémie selon la revendication 1, où :
l'unité d'entrée d'instruction de commande (151) comprend un écran tactile, et
l'unité de génération de signal (153) est prévue pour générer le signal de commande de connexion, lorsque l'instruction de connexion de l'utilisateur est entrée par l'écran tactile.

3. Dispositif à fixation corporelle pour une mesure continue de glycémie selon la revendication 2, où l'unité de génération de signal (153) est prévue pour comparer un motif de l'instruction de connexion entrée par l'écran tactile avec un motif d'ensemble de référence, et, lorsque le motif de l'instruction de connexion est identique au motif d'ensemble de référence, générer le signal de commande de connexion.

4. Dispositif à fixation corporelle pour une mesure continue de glycémie selon la revendication 1, où :
l'unité d'entrée d'instruction de commande (151) comprend un récepteur optique, et
l'unité de génération de signal (153) est prévue pour générer le signal de commande de connexion lorsque l'instruction de connexion de l'utilisateur est entrée par le récepteur optique.

5. Dispositif à fixation corporelle pour une mesure continue de glycémie selon la revendication 4, où l'unité de génération de signal (153) est prévue pour comparer un motif de l'instruction de connexion entrée par le récepteur optique avec un motif d'ensemble de référence, et générer le signal de commande de connexion lorsque le motif de l'instruction de connexion est identique au motif d'ensemble de référence.

6. Dispositif à fixation corporelle pour une mesure continue de glycémie selon la revendication 5, où l'unité de génération de signal (153) est prévue pour générer le signal de commande de connexion par comparaison d'une fréquence et d'un motif de lumière de l'instruction de connexion entrée par le récepteur optique avec une fréquence et un motif de lumière du motif d'ensemble de référence.

7. Dispositif à fixation corporelle pour une mesure continue de glycémie selon la revendication 2 ou la revendication 4, où l'unité de génération de signal (153) est prévue pour rechercher un motif d'ensemble de référence identique à un motif de l'instruction de connexion entrée, et générer le signal de commande de connexion correspondant au motif d'ensemble de référence recherché.

8. Dispositif à fixation corporelle pour une mesure continue de glycémie selon la revendication 7, où le signal de commande de connexion généré par l'unité de génération de signal (153) est un signal de connexion pour une connexion électrique entre la pile (140) et la PCB par l'unité de commutation ou un signal d'interruption pour interrompre la connexion électrique entre la pile (140) et la PCB.

9. Dispositif à fixation corporelle pour une mesure continue de glycémie selon la revendication 2 ou la revendication 4, comprenant en outre un film de blocage empêchant l'entrée de l'instruction de connexion sur l'unité d'entrée d'instruction de commande (151), le film de blocage étant monté de manière amovible sur l'unité d'entrée d'instruction de commande (151).
